# EUROPEAN PATENT SPECIFICATION

(11) **EP 2 696 807 B1**
(45) Date of publication and mention of the grant of the patent: **16.12.2020**
(21) Application number: 12771728.8
(22) Date of filing: 02.04.2012
(51) Int. Cl.: A61F 2/00

(54) **APPARATUS FOR INHIBITING STRESS URINARY INCONTINENCE**
VORRICHTUNG ZUR HEMMUNG STRESSBEDINGTER HARNINKONTINENZ
APPAREIL POUR INHIBER L'INCONTINENCE URINAIRE DE STRESS

(30) Priority: 11.04.2011 US 201161473955 P
(43) Date of publication of application: 19.02.2014
(73) Proprietor: Floelle Inc., Acton, MA 01720 (US)
(72) Inventor: SHAPIRO, Jerrold, M., Maynard, MA 01754 (US); GRANTHAM, Cheri, A., Willis, TX 77318 (US)
(74) Representative: Jones, Graham Henry
(86) International application number: PCT/US2012/031784
(87) International publication number: WO 2012/141923

(56) References cited:
- EP-A1- 0 810 001
- WO-A1-01/26581
- US-A- 4 784 654
- US-A- 5 112 306
- US-A- 6 027 442
- US-A1- 2002 156 342
- US-A1- 2008 269 547
- US-A1- 2009 132 043
- US-A1- 2010 168 656

## Description

### Field

This disclosure relates to devices for abating incontinence in women, and in particular to devices that serve to inhibit or prevent the unwanted discharge of urine from the urinary tract.

### Background

Urinary incontinence, or involuntary urine outflow, affects sixteen percent of women in the United States of America. In about seventy five percent of these women, urinary incontinence occurs when the pressure in their abdomen, called the intra-abdominal pressure, or IAP, rises above a threshold pressure, called IAP1; this is called stress incontinence. In about twenty percent of incontinent women, the detrussor muscle of the urinary bladder involuntarily contracts, producing a sudden urge to urinate; this is called urge incontinence. Women with both stress and urge urinary incontinence are said to have mixed urinary incontinence.

While pharmaceutical treatment is available to relax the detrussor muscle and relieve urge urinary incontinence, there are no pharmaceuticals cleared by the U.S. Food and Drug Administration for treating stress urinary incontinence. Many surgical procedures have been developed to mechanically support the human female urethra or to make it more rigid, but these are expensive, have side effects and are chosen by very few incontinent women. Devices to temporarily block urine flow using a urethral catheter whose proximal end expands inside the bladder need to be replaced every time the woman urinates, and are relatively expensive. Palliative measures, such as wearing an absorbent pad and changing it after every urination, are not only expensive but may leave the skin on the inner thighs wet, leading to abrasions and ulcers; while many pads contain deodorants, the residual urine odor is embarrassing for many incontinent women, leading to restricted social contacts and decreasing their quality of life.

Prior art includes devices inserted through the female urethra into the bladder to seal the bladder neck until the devices are removed, and a device containing a manually operated drainage control valve that is inserted through the female urethra into the bladder as disclosed in U.S. Patent Number 5,234,409.

EP0810001 discloses a manually operated, normally closed, valve that is inserted so that it lies partly in the urinary bladder and partly in the urethra of males and females, including two balloons which can be filled with a liquid.

### Summary

The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes.

The present invention provides apparatus for use within a woman's urethra for inhibiting urine outflow when her intra-abdominal pressure rises, the apparatus having a generally tubular device that has a proximal end and a distal end and defines a longitudinal axis, characterised in that:
the device comprises at least one liquid-filled sealed flexible chamber, where the chambers together are constructed and arranged to define a longitudinal passageway that extends entirely through the device from the proximal end to the distal end; and
wherein the device is constructed and arranged such that an increase in intra-abdominal pressure causes the liquid in the liquid-filled chambers to be displaced from a first state in which the passageway is open along its length and able to conduct urine to a second state in which the passageway is more restricted or is closed off, to inhibit the passage of urine.

The apparatus may automatically inhibit or prevent urinary discharge during sudden elevation of intra-abdominal pressure, which functions without intervention by the user and which does not impede her normal activities.

The apparatus may be a female incontinence device which is automatically activated by increased intra-abdominal pressure. The apparatus may be entirely contained within the body of the user. The apparatus may be easily installed and removed. The apparatus may be operationally fixed within the female urethra. The apparatus may effectively prevent urinary discharge when such discharge is not desired and may permit such discharge when the user desires and the intra-abdominal pressure is less than a certain threshold pressure (IAP2). The apparatus may prevent the growth of bacteria on the surfaces from which the apparatus is manufactured.

Other features of the disclosure will be in part apparent and in part pointed out hereinafter. In accordance with the disclosure, the female incontinence apparatus may define a passageway or conduit having inlet and outlet openings for receiving, conducting and discharging urine. The device may also include one or more structures that obstruct this conduit, but only when the pressure on the urethra surrounding it exceeds a threshold pressure (IAP1). These structures may define a clear path through the apparatus when the pressure on the urethra surrounding it falls below a threshold pressure (IAP2), and the pressure of urine within the conduit exceeds another threshold pressure (IVP1). In one embodiment, the apparatus is located within the urethra and the structures for obstructing the passageway comprise liquid-filled flexible chambers or balloons which are located such that they are partially in contact with the lining of the urethra. Intra-abdominal pressure elevations caused by, for example, coughing or sneezing, are transmitted through the urethra to such chambers, causing the liquid within them to be displaced. The liquid moving within the chambers causes a part of the chambers to obstruct the flow of urine through the passageway. The liquid within each chamber returns to its initial location once intra- abdominal pressure returns to normal and the pressure of urine inside the passageway as a result of micturition exceeds another threshold pressure (IVP2). The apparatus serves as a flow control valve that is normally open, but is automatically closed by increased intra-abdominal pressure, which typically results from the patient coughing, laughing, lifting, sneezing, etcetera. The apparatus thus inhibits or fully prevents involuntary urine leakage. The apparatus may also include structure that holds the passageway in a selected position within the urethra, ideally such that the proximal part of the device (closest to the urinary bladder) which is in contact with the lining of the urethra is also proximal to the part of the urethra that passes through the musculature of the pelvic floor.

In an embodiment of the invention, flexible chambers may extend generally along the longitudinal axis of the device. The chambers may be constructed and arranged to essentially fill the urethra at least at a first location along the longitudinal axis. At the first location, the flexible chambers may each define an essentially circular segment shape such that two or more of them together can essentially fill a hollow cylindrical tubular portion of the device. At a different second location along the longitudinal axis, the device may be constructed and arranged such that the flexible chambers each define an essentially annular segment shape.

The generally tubular device may comprise a frame that has a first tubular section with a first diameter and a second tubular section with a second, larger diameter. The tubular sections may be interconnected. The tubular sections may be interconnected by a spider member. The generally tubular device may further comprise at least two liquid-filled flexible chambers, where the flexible chambers extend generally along the longitudinal axis of the device and are constructed and arranged to essentially fill the urethra at least at the location of the second tubular section. The spider member may comprise a plurality of thin elongated spider members, and there may be an equal plurality of flexible chambers. The flexible chambers may be coupled to the outside of the first tubular section and the inside of the second tubular section. The apparatus may further comprise a retaining ring coupled to the outside of the second tubular section. Proximate the location of the second tubular section, the flexible chambers may each define an essentially circular segment shape. Also, proximate the location of the first tubular section, the flexible chambers may each define an essentially annular segment shape.

### Brief Description of the Drawings

Figures 1a and 1b are end arid side views, respectively, of the frame of an embodiment of an apparatus that can be used to inhibit stress urinary incontinence in human females.
Figures 2a and 2b are longitudinal cross-sectional views of an apparatus that includes the frame shown in figure 1, illustrating a position in which urine can pass and a position in which urine flow is occluded, respectively.
Figure 3 is a perspective view of a second embodiment of an apparatus that can be used to inhibit stress urinary incontinence in human females.
Figure 4 illustrates the performance of a prototype of the apparatus.
Figures 5a-5c are longitudinal cross-sectional views and an end view, respectively, of a third embodiment of the apparatus.
Figures 6-9 illustrate the shapes and arrangements of the chambers for an apparatus that uses two, three, four or five liquid-filled chambers, respectively, with figure "a" showing the relaxed position in which a lumen is formed to allow the passage of urine, and figure "b" showing the closed position in which the lumen is restricted or fully closed along at least a portion of its length, to inhibit urine flow.
Figures 10a, 10b and 10c are views similar to those of figures 5a-5c, for another embodiment that uses only a single chamber.

### Description of Embodiments

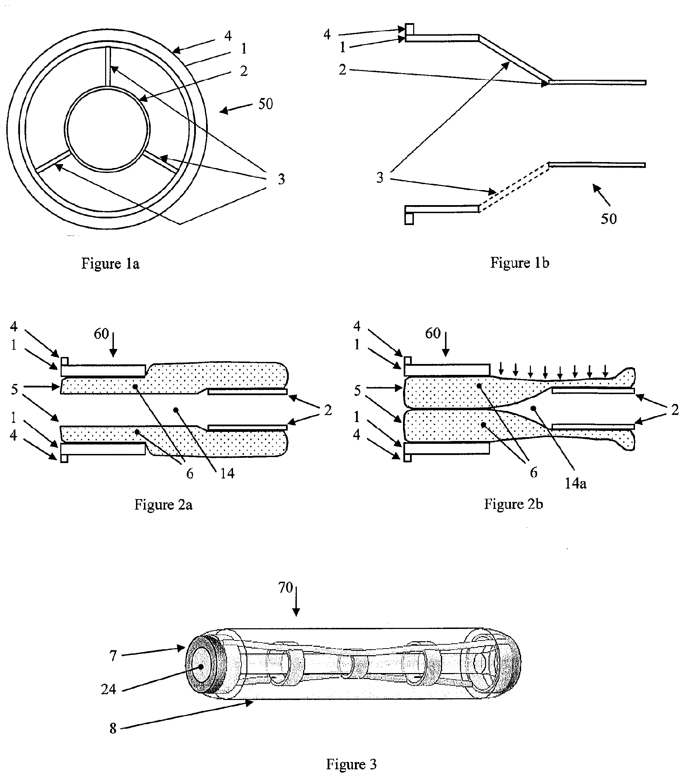

Figure 1 shows the frame 50 of one embodiment of a device 60 that can be used to inhibit or prevent stress urinary incontinence in a human female. Frame 50 consists of an outer tube 1, an inner tube 2, a spider 3 (with the number of vanes equal to the number of elastic chambers or balloons 5) and a retaining ring 4 all connected to form a semi-rigid structure and possibly manufactured as a single piece. Figure 1a is a view of frame 50 perpendicular to its long axis and viewed from its distal end when inserted in the urethra of a woman. Figure 1b is a side view of frame 50. The retaining ring 4 maintains the location of the device along the long axis of the female urethra.

Figure 2 shows the complete device 60. Frame 50 surrounds two or more flexible balloons or chambers 5 (three in this case) which are filled with a fluid 6 that is preferably a biocompatible fluid with low viscosity, such as isotonic saline solution. Any air or other gas has been removed from the balloons and filling fluid so as to render the fluid incompressible. This embodiment includes three balloons, but the apparatus and method herein contemplates the use of one or more balloons or chambers.

The distal end (nearest the vulva when the device is inside the female urethra) of each balloon in this embodiment is generally wedge shaped (more specifically, generally shaped as a circular segment), such that the distal ends of the balloons as a whole occupy a generally circular cylindrical volume. The outer diameter of this cylinder is approximately equal to the inner diameter of the outer tube 1 of frame 50. The rounded part of the distal portion of each balloon 5 (corresponding to the cylinder's circumference) is attached to the inner surface of the outer tube 1 along part or all of their meeting surfaces. The proximal end (nearest the urinary bladder when the device is inside the female urethra) of each balloon is generally shaped like a sector of an annulus, with the inside diameter of such annulus approximately equal to the outer diameter of the inner tube 2 of frame 50 and attached along all or part of their meeting surfaces. Chambers 5 thus are inhibited from moving longitudinally or rotationally relative to frame 50. The spiders 3 that connect the frame's inner and outer tubes pass between the balloons. The outer diameter of the proximal part of each balloon is almost equal to the inner diameter of the frame's outer tube. The outer diameter of the frame's outer tube is approximately equal to the inside diameter of the human adult female urethra.

The volume of fluid inside each balloon is determined by placing a solid cylindrical rod, whose diameter is slightly less than that of the frame's inner tube and whose length exceeds that of the device, inside the frame's inner tube with one end at the device's proximal end and placing a hollow tube of circular cross-section, whose inner diameter is slightly larger than the outside diameter of the frame's outer tube, with one end at the device's proximal end, then filling the multiple balloons with equal amounts of incompressible fluid until all the balloons are full. Once this fill volume is measured and recorded, it will be used to fill each balloon before the balloon is sealed.

When device 60 is placed in the mid-urethra of a woman, and her intra-abdominal pressure, abbreviated IAP, is low, the balloons will have an arbitrary shape. When the woman initiates the micturition reflex, her bladder's detrusor muscles contract. This causes urine to flow from her bladder through her urethra and through the lumen 14 that is located along the longitudinal axis (not shown in the drawing) of device 60, and exit outside her body. Tube 2 maintains a central entrance opening to the device, into which urine flows from the bladder. The fluid pressure of her urine against the balloons in the location within tube 1 pushes the inner portions of the balloons (corresponding to the points of each balloon's circular segment shaped distal end) away from the device's axis. This creates a full-length lumen or passageway 14 for urine flow. This also displaces the fluid inside each balloon from its distal end toward its proximal end, as shown in figure 2a. When the woman is not micturating, but raises her IAP by coughing, laughing, sneezing, jogging, lifting or other means, the raised IAP presses her urethra against the proximal outer surfaces of the balloons above tube 2 (indicated by the arrows in figure 2b). This displaces the fluid within the balloons from their proximal ends toward their distal ends. The movement of fluid causes the distal ends of the balloons to each assume a circular segment shape as shown in figure 2b, thereby occluding the device lumen 14a where it passes through outer frame 1, thus blocking urine flow through the device. Once the IAP returns to normal, the fluid redistributes within each balloon and allows urine flow during micturition.

To prevent the growth of microbes on the surfaces of the device, an antimicrobial substance can be mixed into the materials before they are formed into the balloons and frame. To prevent urine crystals, if any, from adhering to the device all surfaces of the device in contact with urine can be coated with a highly lubricious material.

In a second embodiment, as shown in Fig. 3, the device 70 is fabricated from elastic waterproof material in the shape of a hollow toroid 8 formed from rotating a long rectangle about an axis parallel to and displaced from a long side; a rigid generally hourglass-shaped frame 7 inside the toroid maintains its length and outer diameter at its extremities, while allowing the outer surface of the elastic toroid to be deformed by the adjacent urethra during periods of increased intra-abdominal pressure. The toroid is filled with an incompressible liquid, which may be distilled water, isotonic saline or a fluid whose density and viscosity is equal to or less than that of distilled water at body temperature, 37 degrees Centigrade. When the outer surface of the toroid is pressed inward by the adjacent urethra, the incompressible fluid inside it is displaced and causes the inner surface of the toroid, which normally forms the conduit 24 for urine, to expand into such conduit to narrow the conduit's diameter, greatly increase the conduit's flow resistance (which varies inversely with the fourth power of the conduit diameter) and inhibit or effectively prevent any urine flow through the conduit.

A prototype of this first embodiment consisted of a flexible balloon with an internal channel for urine flow stretched over a rigid plastic frame, as shown in Fig. 3. The device was placed inside a thin-walled elastic tube which simulated the urethra, and whose inner diameter was equal to the outside diameter of the device. Proof of concept testing was performed using a Starling Resistor, which consisted of a pump, the thin-walled elastic tube in an air-tight chamber, and a flow control circuit equipped with a flow meter. The external pressure of the elastic tube, which is the internal pressure of the airtight chamber, was controlled by a rubber bulb and measured by an attached gauge. The flow through the elastic tube was measured by the flow meter. As the average urine flow rate during micturition for females between 14 and 65 years old is between 0.9 and 1.08 L/min, a flow rate of about 1 L/min was used to test the device. The flow registered by the flow meter was recorded as the external pressure on the elastic tube was slowly increased until no flow was registered by the flow meter. This procedure was repeated multiple times.

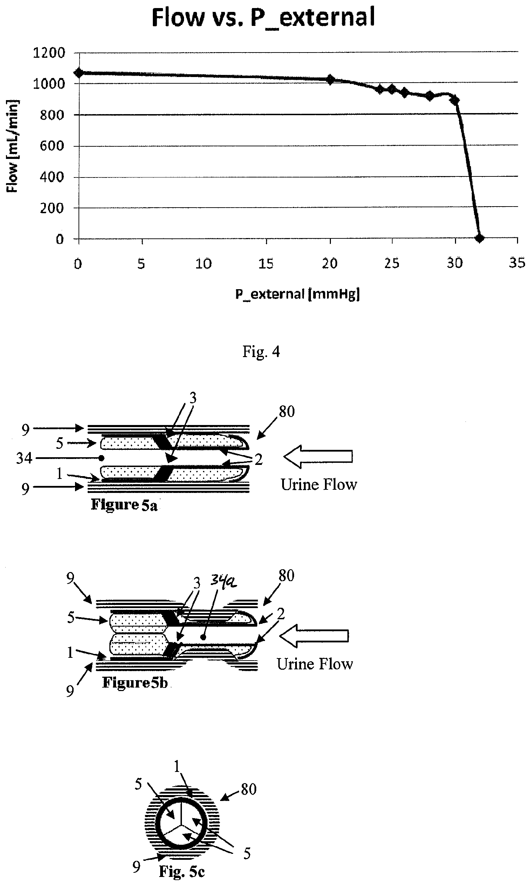

A graph of the average flow rate versus external pressure is shown in Figure 4. As the external pressure on the elastic tube simulating the female urethra was increased, the flow through the embodiment's conduit decreased until it reached zero flow at 32 mmHg, which is 43.5cm H₂O. This is the external pressure that causes occlusion. While this pressure is greater than the specified occlusion pressure of the device, 30 cm H₂O, this value is likely to change when the final device materials are tested. Despite this discrepancy, the results of this test demonstrate that occlusion occurs for physiologic external pressures.

The second embodiment (device 70) can be constructed by connecting together, using adhesives or ultrasonic welding, the ends of a tube of flexible material to form a balloon. We have selected biocompatible materials such as PDMS, a silicone rubber (polydimethylsiloxane), or alternatives like thermoplastic polyurethanes made by Bayer, such as Texin and Desmopan, for the flexible balloon. The more rigid parts of the device can be machined or molded from a biocompatible plastic such as polyetheretherketone (e.g., Zeniva PEEK from Solvay Advanced Polymers LLC in Alpharetta, Georgia) with polysulfone, polyphenylsulfone or polyetherimide as alternative biocompatible plastics. Ionic silver in a zeolite carrier from Agion Technologies in Wakefield, MA, or equivalent antimicrobial compounds, can be incorporated into these flexible and more rigid materials to give them antimicrobial properties.

Once the rigid frame is inserted inside the balloon, the balloon is filled with one of the sterile liquids described above so that air is excluded, and the balloon is sealed. The device looks like that shown in Fig. 3 and has an overall length of about 25 millimeters, and at low intra-abdominal (IAP) and intravesical (IVP) pressures, an outside diameter of about 3 millimeters and a conduit, passageway or lumen 24 whose inside diameter is about 1.5 millimeters. The thickness of the balloon and its mechanical properties can be selected so that when the intra-abdominal pressure is below about 20 cm H₂O, defined as IAP2 above, and the pressure of urine or similar fluid inside the device's conduit is greater than about 40 cm H₂O, defined as IVP1 above, then urine will flow through the device's conduit 24 and allow the patient's bladder to empty. When the intra-abdominal pressure is above about 30 cm H₂O, defined as IAP1 above, and bladder's detrusor muscles are relaxed, then fluid within the device's flexible balloon will transmit the intra-abdominal pressure to the walls of the device's conduit 24, and cause the conduit diameter to become very small, cause its flow resistance to become very high and effectively obstruct flow through such conduit. This will prevent urine leakage during intra-abdominal pressure spikes due to the patient laughing, coughing, sneezing, lifting, etc.

A third embodiment, device 80, is shown in figures 5a, 5b and 5c. As viewed from the urethral meatus (figure 5c), the device includes three flexible and elastic chambers 5 constructed of biocompatible elastomer which may contain an antimicrobial substance, such as silver ions; the device cross section resembles a pie cut into three equal slices as shown in the center of Fig. 5c. The chambers run parallel to the long axis of the urethra for about 25 mm; part of the way from the distal to the proximal end of the device, the chamber cross-section changes to surround a hollow conduit 2 whose diameter is about half that of the about 3 millimeter outside diameter of the device established by portion 1. This proximally located tube connects, via a spider 3 of three vanes passing between the three chambers, to a larger hollow tube 1 that surrounds the outside curved surfaces of the three chambers. Each chamber is filled with sterile isotonic saline solution and sealed; each chamber is filled to a predetermined volume so that, at normal intra-abdominal pressure, a lumen 34 can form between the distal apices of the three chambers to allow the outflow of urine.

In Figs. 5a and 5b, which are not to scale, the striped lines represent the urethra 9; the black lines represent the harder plastic shell comprised of interconnected portions 1, 2 and 3, and the elastic chambers 5 are shown as thin black lines with dots inside to represent the fluid fill. While the urethra of women with stress and mixed incontinence may not be subject to the full amplitude of increases in IAP, the elevated IAP will still exert pressure on the urethral wall, which will be transmitted to the three chambers of the device in the urethral lumen, causing them to move the fluid contained within them from the proximal to the distal end of the device, and to expand that part of the chambers so that they come together to block the device lumen 34a, as shown in Figure 5b. Due to its low viscosity and volume, we expect the fluid inside each balloon to respond quickly to elevated IAP (typically in the range of 85 to 95 cmH₂O) and occlude the urethra during coughs and sneezes. The device will also respond to contraction of the muscular walls of the urethra, part of which contracts under neural control at the start of a sneeze before elevation of the IAP. The elastic chambers' liquid fill volume will be set during the manufacturing process so that the part inside of tube 1 is closed at low IAP and low intra-urethral pressure, but opens at elevated intra-urethral pressure during micturition (typically in the range of 10 to 30 cmH₂O). The flexible balloons can be fabricated using the well-known dip molding technique used to make surgical gloves and condoms. The molds can be made of a water soluble material, so that after the silicone material cures, water can be injected into the part to dissolve and wash out the molding material; the balloon interior can be flushed with a solution that kills bacteria, viruses and fungi, flushed again with sterile water then filled with sterile isotonic saline and sealed. The more rigid parts of the device can be machined or molded from a biocompatible plastic, as described above. Ionic silver in a zeolite carrier can be incorporated into these materials to give them antimicrobial properties, also as described above.

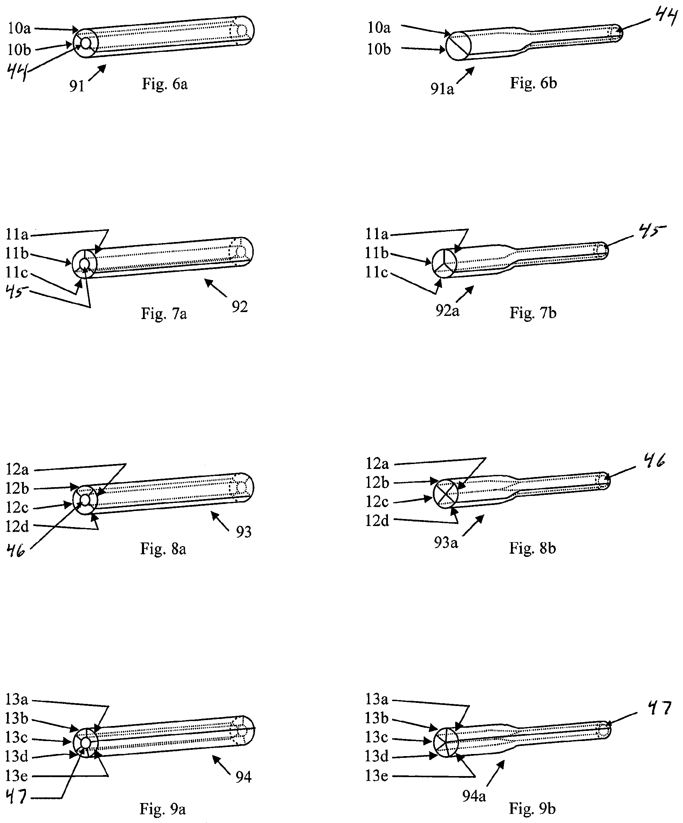

Additional embodiments similar to the second embodiment may be constructed with two, three, four or five (or, conceivably, more if desired) flexible balloons, as shown in figures 6-9, respectively, in which figures 6a-9a show the balloons in their state during micturition, and figures 6b-9b show the same balloons in their state during which the lumen is occluded. These figures show only the balloons and not the frame, which typically includes the spider vanes, an inner tube and an outer tube. The number of vanes typically equals the number of balloons. Figure 6a shows two balloons 10a and 10b that are each generally semi-circular in cross section and can define lumen 44. Figure 7a shows three balloons 11a-c that each generally define a 1/3 circular segment in cross section and can define lumen 45. Figure 8a shows four balloons 12a-d that each generally define a 1/4 circular segment in cross section and can define lumen 46. Figure 9a shows five balloons 13a-e that each generally define a 1/5 circular segment in cross section and can define lumen 47.

All embodiments are inserted into the female urethra inside a short catheter, and held in place while the catheter is withdrawn. As shown in figure 1, a retaining ring can hold the device in place. Alternatively, the device could be attached to the distal urethral mucous membrane via a bioadhesive so that it is held in place and thus is neither expelled nor moves into the bladder. If the device needs to be removed, a wire or other member with a short L-shaped hook at the far end may be inserted through the device's inner tube or lumen until it extends beyond the device's proximal end; rotated, pressed against one side of the inner tube then withdrawn until the hook is against the proximal end of the inner tube; a catheter similar to that used to insert the device is inserted into the urethra and advanced until it separates the device from the urethra, then the catheter, device and wire are simultaneously withdrawn.

The embodiments describe the use of circular sector-shaped chamber segments that co-act to close the device lumen when the intra-abdominal pressure rises. However, the apparatus could use other means to channel fluid flow caused by a constriction of the urethra due to an IAP rise. For example, there could be one or more chambers or balloons used, and the chambers could have more arbitrary shapes, so long as the chamber or the multiple chambers fill or essentially fill one location of the inside of the device tube when fluid inside the chamber(s) is moved by the increased IAP.

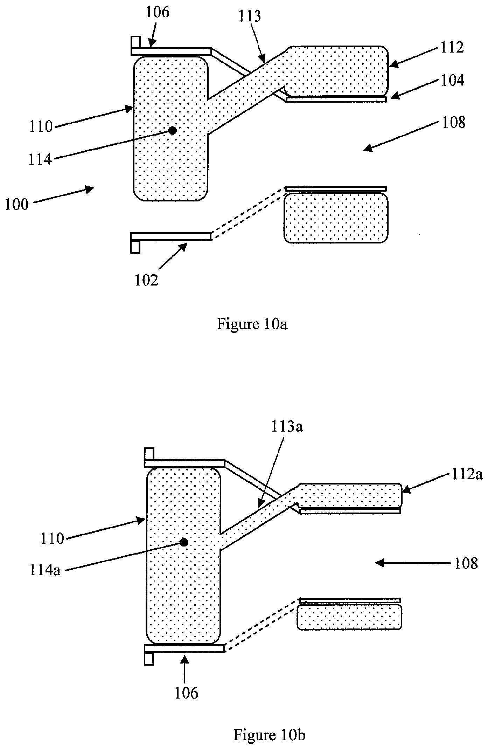

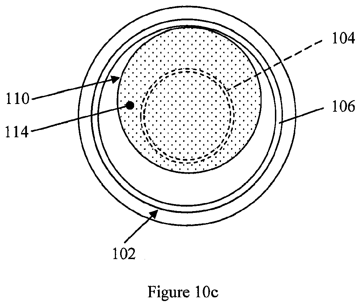

As just one example, there could be a single spherical or cylindrical balloon inside the lumen of the larger frame so that when it is full it touches the inside of the frame (i.e., fills the frame) to block flow, but when it is not full, urine can flow around the outside of the balloon between the balloon and the frame around some or all of the balloon's circumference. A small section of the balloon's circumference could be attached to the inner surface of the frame to prevent the balloon from getting twisted. This design would retain the rigid frame described above, and have the proximal part of the balloon surround most of the inner tube, but with a single connection between the proximal and distal parts of the balloon. An example is shown in figures 10a, 10b and 10c. Apparatus 100 includes frame 102 of the type described above, with outer tube 106 longitudinally displaced from inner tube 104. A single chamber or balloon 110 comprises portion 112 fixed to the outside of tube 104 and portion 114 located within tube 106 and fixed to tube 106 in one location, as shown in the end view of figure 10c. Integral connecting portion 113 allows fluid flow between portions 112 and 114. In the "open" position shown in figures 10a and 10c, lumen 108 is open along the entire length of the apparatus. When portion 112 is squeezed by the urethra it collapses into form 112a; this pushes fluid through connecting portion 113a and into portion 114, to create enlarged portion 114a which fills the lumen within tube 106. This blocks urine flow through apparatus 100. The volume of fluid to be placed in the single balloon could be determined by placing a crescent shaped object between the balloon 110 and the outer tube 106 in addition to putting the part 106 inside a cylinder whose inside diameter is slightly larger than that of part 106 and whose length is a least as long as the device along its longitudinal axis, and then inflating the balloon to slightly above local atmospheric pressure.

## Claims

1. An apparatus for use within a woman's urethra (9) for inhibiting urine outflow when her intra-abdominal pressure rises, the apparatus having a generally tubular device (60) that has a proximal end and a distal end and defines a longitudinal axis, **characterized in that**:
the device (60) comprises at least two liquid-filled sealed flexible chambers (5), wherein the chambers (5) together are constructed and arranged to define a longitudinal passageway (14) that extends entirely through the device (60) from the proximal end to the distal end; and
wherein the device (60) is constructed and arranged such that an increase in intra-abdominal pressure causes the liquid (6) in the liquid-filled chambers (5) to be displaced from a first state in which the passageway (14) is open along its length and able to conduct urine to a second state in which the passageway (14) is more restricted or is closed off, to inhibit the passage of urine.

2. The apparatus of claim 1 wherein the flexible chambers (5) lie along the longitudinal axis of the device (60) and are constructed and arranged to essentially fill the urethra (9) at least at a first location along the longitudinal axis.

3. The apparatus of claim 2 wherein at the first location the flexible chambers (5) each define an essentially circular segment shape and/or wherein at a different, second location along the longitudinal axis the device (60) is constructed and arranged such that the flexible chambers (5) each define an essentially annular segment shape.

4. The apparatus of claim 1 wherein the device (60) comprises a frame (50) comprising a first tubular section (2) with a first diameter and a second tubular section (1) with a second, larger diameter.

5. The apparatus of claim 4 wherein the tubular sections (1, 2) are interconnected.

6. The apparatus of claim 5 wherein the tubular sections (1, 2) are interconnected by a spider member (3).

7. The apparatus of claim 6 wherein the device (60) comprises at least two liquid-filled sealed flexible chambers (5), where the flexible chambers (5) lie along the longitudinal axis of the device (60) and are constructed and arranged to essentially fill the urethra (9) at least at the location of the second tubular section (1).

8. The apparatus of claim 7 wherein the spider (3) comprises a plurality of thin elongated spider members, and there are an equal plurality of flexible chambers (5):

9. The apparatus of claim 8 wherein the flexible chambers (5) are coupled to the outside of the first tubular section (2) and the inside of the second tubular section (1).

10. The apparatus of claim 9 further comprising a retaining ring (4) coupled to the outside of the second tubular section (1).

11. The apparatus of claim 10 wherein proximate the location of the second tubular section (1) the flexible chambers (5) each define an essentially circular segment shape and/or wherein proximate the location of the first tubular section (2) the flexible chambers (5) each define an essentially annular segment shape.

## Patentansprüche

1. Apparat zur Verwendung in der Urethra (9) einer Frau zum Hemmen des Herausfließens von Harn, wenn ihr intraabdominaler Druck steigt, wobei der Apparat eine allgemein rohrförmige Vorrichtung (60) aufweist, die ein proximales Ende und ein distales Ende aufweist und eine Längsachse definiert, **dadurch gekennzeichnet, dass**:
die Vorrichtung (60) mindestens zwei flüssigkeitsgefüllte versiegelte flexible Kammern (5) umfasst, wobei die Kammern (5) zusammen so konstruiert und angeordnet sind, dass sie einen längsgerichteten Durchgang (14) definieren, der sich ganz durch die Vorrichtung (60) hindurch von dem proximalen Ende zu dem distalen Ende erstreckt; und
wobei die Vorrichtung (60) so konstruiert und angeordnet ist, dass ein Anstieg des intraabdominalen Drucks bewirkt, dass die Flüssigkeit (6) in den flüssigkeitsgefüllten Kammern (5) von einem ersten Zustand, in dem der Durchgang (14) entlang seiner Länge offen ist und in der Lage ist, Harn hindurch zu leiten, zu einem zweiten Zustand verschoben wird, in welchem der Durchgang (14) stärker eingeengt oder abgesperrt ist, um die Passage von Harn zu hemmen.

2. Apparat nach Anspruch 1, wobei die flexiblen Kammern (5) entlang der Längsachse der Vorrichtung (60) liegen und so konstruiert und angeordnet sind, dass die Urethra (9) mindestens an einer ersten Stelle entlang der Längsachse im Wesentlichen gefüllt ist.

3. Apparat nach Anspruch 2, wobei an der ersten Stelle die flexiblen Kammern (5) jeweils eine im Wesentlichen kreisförmige Segmentform definieren, und/oder wobei die Vorrichtung (60) an einer anderen zweiten Stelle entlang der Längsachse so konstruiert und angeordnet ist, dass die flexiblen Kammern (5) jeweils eine im Wesentlichen ringförmige Segmentform definieren.

4. Apparat nach Anspruch 1, wobei die Vorrichtung (60) einen Rahmen (50) umfasst, der einen ersten rohrförmigen Abschnitt (2) mit einem ersten Durchmesser und einen zweiten rohrförmigen Abschnitt (1) mit einem zweiten größeren Durchmesser umfasst.

5. Apparat nach Anspruch 4, wobei die rohrförmigen Abschnitte (1, 2) miteinander verbunden sind.

6. Apparat nach Anspruch 5, wobei die rohrförmigen Abschnitte (1, 2) über ein Spinnenelement (3) miteinander verbunden sind.

7. Apparat nach Anspruch 6, wobei die Vorrichtung (60) mindestens zwei flüssigkeitsgefüllte versiegelte flexible Kammern (5) umfasst, wobei die flexiblen Kammern (5) entlang der Längsachse der Vorrichtung (60) liegen und so konstruiert und angeordnet sind, dass die Urethra (9) an mindestens der Stelle des zweiten rohrförmigen Abschnitts (1) im Wesentlichen gefüllt ist.

8. Apparat nach Anspruch 7, wobei die Spinne (3) eine Vielzahl von dünnen länglichen Spinnenelementen umfasst und es eine gleiche Vielzahl von flexiblen Kammern (5) gibt.

9. Apparat nach Anspruch 8, wobei die flexiblen Kammern (5) an die Außenseite des ersten rohrförmigen Abschnitts (2) und die Innenseite des zweiten rohrförmigen Abschnitts (1) gekoppelt sind.

10. Apparat nach Anspruch 9, des Weiteren umfassend einen Haltering (4), der an die Außenseite des zweiten rohrförmigen Abschnitts (1) gekoppelt ist.

11. Apparat nach Anspruch 10, wobei nahe der Stelle des zweiten rohrförmigen Abschnitts (1) die flexiblen Kammern (5) jeweils eine im Wesentlichen kreisförmige Segmentform definieren, und/oder wobei nahe der Stelle des ersten rohrförmigen Abschnitts (2) die flexiblen Kammern (5) jeweils eine im Wesentlichen ringförmige Segmentform definieren.

## Revendications

1. Appareil destiné à être utilisé à l'intérieur de l'urètre (9) d'une femme pour empêcher un écoulement d'urine lorsque sa pression intra-abdominale augmente, l'appareil comportant un dispositif généralement tubulaire (60) qui possède une extrémité proximale et une extrémité distale et définit un axe longitudinal, **caractérisé en ce que** :
le dispositif (60) comprend au moins deux chambres flexibles hermétiques remplies de liquide (5), dans lequel les chambres (5) sont construites et disposées ensemble pour définir un passage longitudinal (14) qui s'étend entièrement à travers le dispositif (60) depuis l'extrémité proximale vers l'extrémité distale ; et
dans lequel le dispositif (60) est construit et disposé de sorte qu'une augmentation de pression intra-abdominale amène le liquide (6) dans les chambres remplies de liquide (5) à être déplacé d'un premier état dans lequel le passage (14) est ouvert sur sa longueur et peut conduire l'urine à un second état dans lequel le passage (14) est plus resserré ou est refermé, pour empêcher le passage d'urine.

2. Appareil selon la revendication 1, dans lequel les chambres flexibles (5) reposent le long de l'axe longitudinal du dispositif (60) et sont construites et disposées pour essentiellement remplir l'urètre (9) au moins à un premier emplacement le long de l'axe longitudinal.

3. Appareil selon la revendication 2, dans lequel, au premier emplacement, les chambres flexibles (5) définissent chacune une forme de segment essentiellement circulaire et/ou dans lequel, à un second emplacement différent le long de l'axe longitudinal, le dispositif (60) est construit et disposé de sorte que les chambres flexibles (5) définissent chacune une forme de segment essentiellement annulaire.

4. Appareil selon la revendication 1, dans lequel le dispositif (60) comprend un cadre (50) comprenant une première section tubulaire (2) ayant un premier diamètre et une seconde section tubulaire (1) ayant un second diamètre plus grand.

5. Appareil selon la revendication 4, dans lequel les sections tubulaires (1, 2) sont interconnectées.

6. Appareil selon la revendication 5, dans lequel les sections tubulaires (1, 2) sont interconnectées par un élément en étoile (3).

7. Appareil selon la revendication 6, dans lequel le dispositif (60) comprend au moins deux chambres flexibles hermétiques remplies de liquide (5), les chambres flexibles (5) reposant le long de l'axe longitudinal du dispositif (60) et étant construites et disposées pour essentiellement remplir l'urètre (9) au moins à emplacement de la seconde section tubulaire (1).

8. Appareil selon la revendication 7, dans lequel l'élément en étoile (3) comprend une pluralité de segments d'élément en étoile allongés minces, et le nombre de pluralités de chambres flexibles (5) est identique.

9. Appareil selon la revendication 8, dans lequel les chambres flexibles (5) sont accouplées à l'extérieur de la première section tubulaire (2) et à l'intérieur de la seconde section tubulaire (1).

10. Appareil selon la revendication 9, comprenant en outre un anneau de retenue (4) accouplé à l'extérieur de la seconde section tubulaire (1).

11. Appareil selon la revendication 10, dans lequel, à proximité de l'emplacement de la seconde section tubulaire (1), les chambres flexibles (5) définissent chacune une forme de segment essentiellement circulaire et/ou dans lequel, à proximité de l'emplacement de la première section tubulaire (2), les chambres flexibles (5) définissent chacune une forme de segment essentiellement annulaire.
